# EUROPEAN PATENT APPLICATION

(11) **EP 3 939 597 A1**
(43) Date of publication of application: **19.01.2022**
(21) Application number: 20186334.7
(22) Date of filing: 16.07.2020
(51) Int. Cl.: A61K 31/731, A61P 31/14

(54) **ANTIVIRAL PHARMACEUTICAL COMPOSITION COMPRISING A CARRAGEENAN COMPONENT**

(71) Applicant: Marinomed Biotech AG, 2100 Korneuburg (AT)
(72) Inventor: The designation of the inventor has not yet been filed
(74) Representative: Bogensberger, Burkhard

(57) **Abstract**

The present invention relates to carrageenan for use as an antiviral active ingredient in an antiviral effective amount in a pharmaceutical composition or medicament for the prophylactic or therapeutic intervention of a SARS-CoV-2 infection in a human individual.

## Description

### FIELD OF TECHNOLOGY

The present invention is in the fields of biochemistry, virology and medicine and relates to sulfated polysaccharides, and particularly to carrageenans, in the manufacture of an antiviral pharmaceutical composition or medicament for the prophylactic or therapeutic treatment of SARS-CoV-2-infection.

### BACKGROUND

The coronaviridae family includes coronavirus and torovirus. Coronaviruses are known to infect the upper respiratory tract and the gastrointestinal tract in mammals and birds. It is believed that coronaviruses cause a high percentage of all common colds in human adults.

WO2009/027057 discloses the use of fucoidans and carrageenans in the prophylactic and therapeutic treatment of various respiratory viral infections caused by members of the paramyxoviridae, orthomyxoviridae, adenoviridae and/or coronaviridae families. More precisely, WO2009/027057 *inter alia* reports the results of a coronavirus induced cell death inhibition experiment in cat kidney (CK) cells wherein the CK cells were infected with feline coronavirus FIP in the presence or absence of iota-, kappa- or lambda-carrageenan. It is further disclosed therein that all three types of carrageenan (i.e. iota-, kappa- and lambda-carrageenan) inhibit the coronavirus mediated cell death in CK cells at the highest concentration of 400 *µ*g/ml and that iota-carrageenan showed significant inhibition even at a concentration as low as 4 *µ*g/m), while kappa- and lambda-carrageenan were not effective at this concentration. It was concluded from the results that the carrageenan inhibitory effect on host cell penetration by corona virus was due to reasons other than a chemical or structural modification of the coronavirus-specific receptor(s). It was also found that the pretreatment of the host cells with carrageenan prior to infection did not significantly improve cell protecton against coronavirus infection. The results suggested that in order to achieve a significant protection against coronavirus infection the carrageenan component acting as an antiviral agent must be present at the time of infection, i.e. when an interaction between the virus and the host cell is about to occur.

Sulphated polysaccharides including carrageenans and fucoidan have been known for their antiviral efficacy for decades. In a most interesting review, Gonzalez M.E. et al. (1987, Antimicrob. Agents Chemother. 31, 1388-1393) report an antiviral efficacy of different sulphated polysaccharides including iota-carrageenan against several animal viruses. Iota-carrageenan showed antiviral activity against the enveloped viruses HSV-1, HSV-2, Semliki Forest virus (SFV), vaccinia virus and African swine fever virus (ASF) and against the naked encephalomyocarditis (EMC) virus. Iota-carrageenan had no effect on the enveloped viruses vesicular stomatitis virus (VSV) and measles virus and on the naked viruses polio virus type 1 and adenovirus type 5.

In view of differing experiences made and further in view of sometimes even contradictory results obtained by researches in their attempts of antiviral treatment of varying viral species with carrageenan the authors of US 5,658,893 summarized at column 2, last paragraph bridging over to col. 3, that *"in view of the different responses by different viruses to sulphated polysaccharides, it is clear that the response of a particular virus to carrageenan cannot be predicted with certainty without experimentation. The mechanism by which sulphated polysaccharides, particularly the carrageenans, inhibit viral replication and infectivity may not be uniform as different investigators reported contradictory findings when working with different viruses and cell types. It would **not** be obvious to one skilled in the art that a substance such as a sulphated polysaccharide that is an effective inhibitor of one virus would demonstrate similar efficacy against another virus."*

This situation has not changed over the years and is still applicable.

### BRIEF DESCRIPTION OF THE INVENTION

The challenging worldwide spreading pandemic coronavirus disease 2019 (COVID-19) is caused by a virus that has been denominated Severe Acute Respiratory Syndrome Coronavirus 2 (SARS-CoV-2). Before entering the cell the virus attaches to the host cell surface. It has been found that the viral spike protein recognizes and attaches to the ACE2-receptor (Angiotensin-Converting Enzyme 2 receptor) located on the surface of type I and II pneumocytes, endothelial cells, and ciliated bronchial epithelial cells. It is therefore very likely that drugs interfering with the interaction between the spike protein of SARS-CoV-2 and the ACE2 receptor may offer protection against infection by this kind of virus.

Based on this recognition of the pertinent art and further in view of own in-depth knowledge of the characteristics and possible antiviral efficacy of carrageenans the present inventors have undertaken to find out whether carrageenan could also be effectively used in the prophylactic or therapeutic intervention of SARS-CoV-2 infections in mammals.

In order to accomplish this goal a suitable in vitro testing setup was used for infecting eukaryotic target cells with a commercially available modified lentivirus mimicking part of the outer surface of SARS-CoV2 and further bearing a luciferase reporter gene construct for facilitating analytical determination of infection rates.

It is an objective of the present invention to provide a useful carrageenan-based antiviral composition or medicament for the prophylactic intervention of an infection, i.e. for reducing the risk of infection, of mammals including humans and non-human animals, by SARS-CoV-2.

It is a further objective of the present invention to provide a useful carrageenan-based antiviral composition or medicament for the therapeutic treatment of SARS-CoV-2 infected individuals.

It is particularly envisaged to provide useful medication for individuals especially susceptible to or at increased risk of coronavirus infection including high-risk patients selected from the group consisting of COPD-patients, asthma patients, individuals suffering from allergies, or from impaired immune systems, from impaired cardiac systems, or from impaired pulmonary systems, and transplantation patients.

### FIGURES

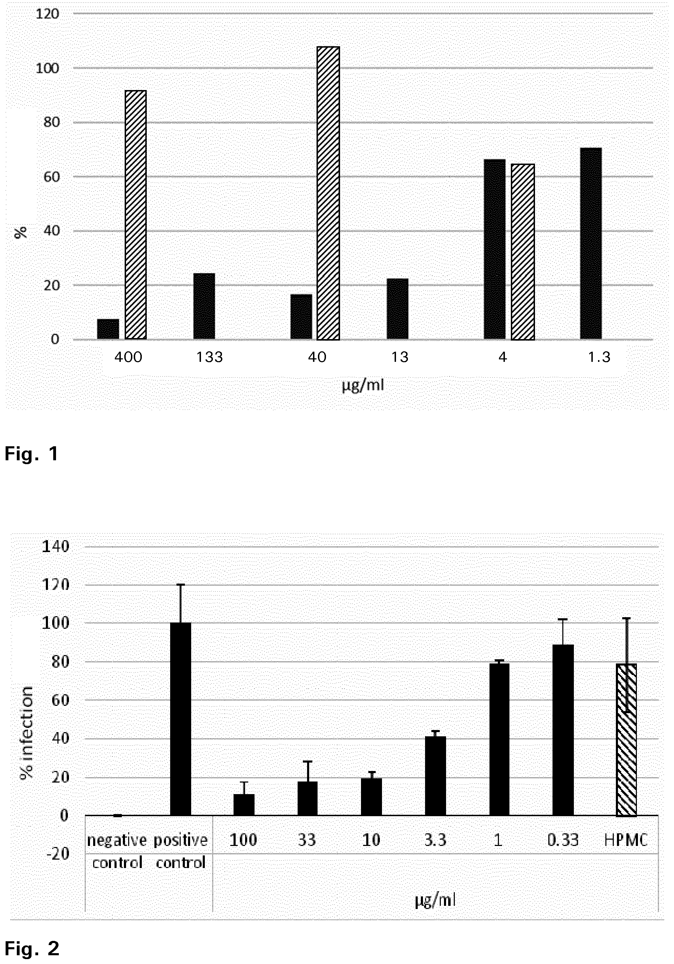

Fig. 1 discloses the results of a neutralization assay demonstrating the inhibitory efficacy of iota-carrageenan and CMC at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment); x-axis = varying concentrations of iota-carrageenan and CMC in ug/ml; black bars = iota carrageenan; hatched bars = CMC.
Fig. 2 discloses the results of a neutralization assay demonstrating the inhibitory efficacy of iota-carrageenan and HPMC at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment) and negative control ( = 0% infection; mock-infected cells); x-axis = varying concentrations of iota-carrageenan and HPMC in ug/ml; black bars = iota carrageenan; hatched bar = HPMC at 100 ug/ml.

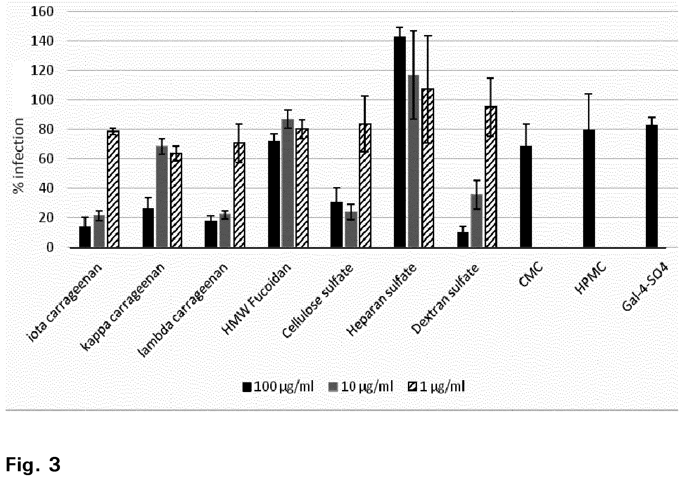

Fig. 3 discloses the results of a neutralization assay demonstrating the inhibitory efficacy of various sulfated polymers at varying concentrations on the infection of ACE2-HEK293 cells with SARS-CoV-2 spike glycoprotein pseudotyped lentivirus bearing a luciferase reporter gene; y-axis = % infected cells relative to positive control (positive control = 100%; number of infected cells without carrageenan or CMC treatment); x-axis = various polymers tested at 3 different concentrations, i.e. 100ug/ml (black bars); 10ug/ml (grey bars); 1ug/ml (hatched bars); CMC, HPMC and Gal-4-SO4 at 100ug/ml.

### DETAILED DESCRIPTION OF THE INVENTION

The term "antiviral active ingredient" as used herein refers to a compound that is effective in directly or indirectly interfering with at least one viral action selected from the group consisting of virus penetration of eukaryotic cells, virus replication in eukaryotic cells, virus assembly, virus release from infected eukaryotic cells, or that is effective in unspecifically inhibiting a virus titer increase or in unspecifically reducing a virus titer level in a eukaryotic or mammalian host system. It also refers to a compound that prevents from or reduces the likelihood or risk of getting a viral infection.

The present pharmaceutical composition may be administered, as the case may be, before or after the onset of a viral infection, i.e. for prophylactic or therapeutic treatment purposes, or for both prophylactic and therapeutic administration.

The terms "prophylaxis", "prophylactic intervention" or "prophylactic treatment" as used herein relate to the administration of the present pharmaceutical composition to a healthy individual in order to reduce said individual's susceptibility for a viral infection with coronavirus.

The term "therapy", "therapeutic intervention" or "therapeutic treatment" as used herein relates to the administration of the present pharmaceutical composition in order to achieve a reduction in severity and/or frequency of symptoms, elimination of symptoms and/or underlying cause, prevention of the occurrence of symptoms and/or their underlying cause, and/or improvement or remediation of damage directly caused by or indirectly associated, e.g. through secondary infection, with a viral infection by coronavirus, especially SARS-CoV-2.

It is known that orthomyxo- and paramyxoviruses and several other viruses attach to the host cell via binding to receptors present on the host cell surface, the receptors typically being glycoproteins or glycolipids containing sugar residues including sialic acid (N-actyl neuramic acid) and heparan sulphate residues.

In earlier *in vitro* studies it was observed by the present inventors that exposing host cells to carrageenan, in particular iota- and/or kappa-carrageenan, prior to infection may alter the structure of the cell surface of a said host cell, presumably by causing conformational changes in the three dimensional structure of certain cell surface receptors or by masking and/or modifying said receptors. Yet, a protective effect against coronavirus infection was only achieved in the presence of carrageenan. It was therefore concluded that with coronavirus the antiviral effect may be due to attachment of the carrageenan polymer to the virions rather than to chemical or physical interaction of the polymer with the host cell receptors.

Carrageenan is a generic term for linear sulphated galactose-based polysaccharides extracted from seaweed (rhodophyceae). There exist more than 10 structurally different carrageenans, their nature depending on the seaweed genus from which they are extracted. The three main types are iota-, kappa- and lambda-carrageenan, which differ slightly in their structure and degree of sulphatation. Iota-carrageenan is a soft-gel forming sulphated galactan predominantly extracted from red seaweed *Gigartina stellata* and *Chondrus crispus.* Kappa-carrageenan yields strong, rigid gels and is predominantly obtained from *Kappaphycus cottonii.* Lambda-carrageenan, which is the most common form, is frequently used to thicken dairy products.

Carrageenan useful for topical application to the mucosa in accordance with the present invention has a molecular weight ranging from about 15,000 to 5,000,000 Da, wherein fractions having average molecular weights of more than 50,000 Da, and especially fractions having average molecular weights in the range of from 50,000 to 3,000,000 Da are particularly preferred.

Fucoidan is a sulphated polysaccharide mainly extracted from various species of brown seaweed, typically from *Undaria pinnatifida.* There are two types of pharmacological grade fucoidans available on the market, a high molecular weight fucoidan (HMWF) fraction having an average molecular weight ranging from about 1,000,000 to 2,000,000 Da (e.g. Kraeber, Germany) and a low molecular weight fucoidan (LMWF) fraction having an average molecular weight of 8,200 Da.

F-fucoidan is mainly composed of sulphated esters of fucose, while U-fucoidan is comprised of about 20 % of glucuronic acid.

Systemic, e.g. parenteral or oral administration is possible, too, especially using lower molecular weight polymers having an average molecular weight in the 15,000 to 100,000 Da range.

The pharmaceutical composition according to the present invention adjusted for parenteral use may be provided as a preparation selected from the group of skin lotions, creams, ointments, gels, powders including powders for inhalation, solutions including solutions for inhalation, sprays, foams, liquid drops or gargle solutions. The carrageenan preparations may, however also be adjusted for oral administration, e.g. as liquid solutions, or semi-solid or solid preparations such as dry powders, tablets, capsules, dragees or any other orally ingestible galenic form.

Where the composition is for topical use and is liquid or semi-solid it typically comprises in its ready-for-use form carrageenan in an amount of between 0.01 and 10 %, preferably between 0.01 and 5 %, most preferably between 0.1 and 2 % by weight, relativ to the total volume of the composition. Where the composition is solid it typically comprises in its ready-for-use form carrageenan in an amount of between 0.01 and 10 %, preferably between 0.01 and 5 %, most preferably between 0.1 and 2 % by weight relative to the total weight of the composition.

If not indicated otherwise the term "carrageenan" as used herein refers to either iota-, or kappa-, or lambda-carrageenan, or to a mixture of at least two of these carrageenan variants.

The carrageenan of the present invention can further be a homopolymer or a heteropolymer. A carrageenan homopolymer is built of subunits of only one kind of either iota-, or kappa-, or lambda-carrageenan, whereas a carrageenan heteropolymer comprises subunits of at least two of said carrageenans, typically of iota- and kappa-carrageenan.

A "mixture" of carrageenans may thus refer to a composition of matter comprising a mixture of at least two of iota-, kappa-, and lambda-carrageenans but also to a composition of matter comprising carrageenan heteropolymers, the latter optionally together with at least one of the corresponding carrageenan homopolymers.

Typically, the antiviral pharmaceutical compositions according to the present invention are substantially free of carrageenans other than iota-, kappa-, and lambda-carrageenan, i.e. comprise either iota-, kappa-, or lambda carrageenan, or any mixture of these carrageenans, in an amount of 80 % or more, preferably of 90 % or more, and especially of up to 99 % (w/w) or more, relative to the dry weight of all carrageenans present in the composition.

For some applications the composition is substantially comprised solely of iota-carrageen, while for other applications the composition comprises more than 50 %, preferably more than 70 %, and especially up to more than 95 % (w/w) by dry weight of iota-carrageenan, relative to the total dry weight of all carrageenans present in the composition.

The antiviral carrageenan compositions in accordance with the present invention may further comprise at least one pharmaceutically acceptable carrier and/or additive suitable and permitted for medical application.

The carrier may be a diluent, e.g. water, saline, optionally phosphate buffered saline (PBS), an excipient, or another vehicle which facilitates the administration of the composition. Where the composition is solid, semi-solid or fluid the groups of carriers and additives, respectively, may comprise but are not limited to SiO₂, TiO₂, a binder such as microcrystalline cellulose, polyvinylpyrrolidone, gum tragacanth, gelatine, starch, lactose, lactose monohydrate, alginic acid or maize starch; a lubricant or surfactant like magnesium stearate or sodium lauryl sulphate; a glidant like e.g. colloidal silicon dioxide; a sweetening agent such as sucrose or saccharin.

Still further additives may be selected from the group consisting of physiologically acceptable preservatives, pharmaceutically acceptable alkali metal salts like sodium, potassium, lithium or ammonium chlorides, buffers or pH adjusting agents such as citric acid, acetic acid, fumaric acid, hydrochloric acid, malic acid, nitric acid, phosphoric acid, propionic acid, sulphuric acid and tartaric acid, and combinations of said acids.

For many applications it is useful that the composition comprises sodium chloride as an additive.

Typically, a pharmaceutically acceptable salt is present in the composition in an amount of not more than 1 %, preferably not more than 0.6 % (w/v). Salt concentrations beyond 1.5% would impair the carrageenan's antiviral efficacy.

In the compositions of the present invention the carrageenan itself may be present as a salt, too, preferably as a sodium salt.

It is preferred that the present compositions for prophylactic or therapeutic medical application be provided as sterile, germ-free, pathogen-free, pyrogen-free and/or allergen-free preparations.

Carrageenan was found to be non-toxic upon oral or dermal administration, or upon inhalation, even when applied at extremely high doses. It was therefore classified as "generally recognized as safe" (GRAS) by the Food and Drug Administration (FDA).

It is also within the scope of the present invention to use a carrageenan component comprising iota-, kappa-, or lambda-carrageenan, or any mixture of said carrageenans, most preferably iota-carrageenan, as an antiviral agent in the manufacture of pharmaceuticl compositions or medicaments, wherein the carrageenan component is present in an antiviral effective amount in combination with at least one specific drug usually applied in the prophylaxis or therapy of infectious and/or inflammatory diseases, allergies and/or conditions of an impaired or suppressed immune system.

It is preferred, however, that the carrageenan component comprising iota-, or kappa-, or lambda-carrageenan, or any mixture of the foregoing carrageenans, is the sole or predominant active antiviral ingredient in compositions for the prophylactic or therapeutic intervention of coronavirus infections, including SARS-CoV-2 infections, according to the present invention.

Parenteral administration of the pharmaceutical composition according to the present invention is preferred, particularly administration in the form of a nasal spray, a mouth spray, a liquid solution for inhalation, a powder for inhalation, a foam, liquid drops or gargle solutions. Nasal and mouth sprays as well as foams and gargle solutions are particularly useful in the prophylaxis of coronavirus infection, wheras preparations for inhalation as well as gargle solutions are particularly useful in the therapeutic treatment of infected individuals.

Inhalation of iota-carrageenan based preparations in accordance with the present invention may substantially alleviate the severe respiratory symptoms caused by SARS-CoV-2 observed with many COVID-19 patients and especially with high-risk patients, i.e. patients selected from the group of COPD-patients, asthma patients, individuals suffering from allergies, or from impaired immune systems, from impaired cardiac systems, or from impaired pulmonary systems, and transplantation patients.

In addition, the present pharmaceutical composition may be applied, e.g. sprayed - much like a sanitizer or disinfectant - onto solid surfaces including hands, fingers, gloves, hygiene tissues or papers including nasal tissues or papers, in order to exert a virucide effect at least to some extent, thus contributing to reducing an individual's repeated self-infection by contaminated fingertips and also to reduce viral distribution among different individuals that are in close, e.g. hand-to-hand, contact with each other.

Further items coated, impregnated or soaked with a pharmaceutical composition on a iota-carrageenan basis comprise cotton swabs, dust masks or sanitary or medical facial masks. Even lipsticks can be formulated to contain an antiviral effective amount of iota-carrageenan. These hygiene or sanitation articles can be used prophylactically or for therapeutical treatment against a coronavirus infection and may assist in the prevention or reduction of a risk of infection.

It is known in the art that attachment and entry of SARS-CoV-2 is mediated by the spike glycoprotein (SGP). In addition to its interaction with its receptor, i.e. human angiotensin converting enzyme 2 (ACE2), the spike glyoprotein has been found to also bind to glycosaminoglycans like heparan sulfate, which is found on the surface of virtually all mammalian cells.

The following examples confirm a strong inhibitory effect of the carrageenan polymers on the infection of ACE2-receptor bearing mammalian cells in a SARS-CoV-2 Spike Pseudotyped Lentivirus assay.

It may be inferred from the strong infection inhibitory effect of the iota-carrageenan polymers that the intervention of the polymers with the SGP-driven entry of the pseudotyped lentivirus into the mammalian target cells is mediated by both the negative charges of the sulphated galactose-based polymers and by certain structural relationships between the polymers on one hand and the ACE2 receptor of the target cell and/or the spike glycoprotein of the virus on the other hand.

### EXAMPLE 1: Inhibitory Effect of Iota-Carrageenan on SARS-CoV2 Infection

The inhibitory effect of iota-carrageenan on SARS-CoV2 infection has been determined in an in vitro neutralization assay based on the commercially available SARS-CoV-2 spike pseudotyped lentivirus assay (BPS Bioscience, Inc., Catalog # 79942; URL: https://bpsbioscience.com/spike-sars-cov-2-pseudotyped-lentivirus-luc-reporter-79942). Using the modified lentivirus system allows for measuring the transduction-inhibitory activity of antiviral agents against SARS-CoV-2 in a biosafety level 2 facility.

According to the manufacturer's data sheet the SARS-CoV-2 Spike Pseudotyped Lentivirus comprises a SARS-CoV-2 Spike (Genbank Accession #QHD43416.1) as the envelope glycoproteins. In addition, these pseudovirions also contain the firefly luciferase gene driven by a CMV promoter. Therefore, the spike-mediated cell entry (= transduction equivalent to infection) can be measured via luciferase reporter activity.

### Experimental design

Incubation media were used according to the assay manufacturer's recommendations, i.e. HEK293 growth medium MEM with 10% FBS, 0.1 mM nonessential amino acids, 1 mM sodium pyruvate.

In a 96-well-plate approximately 7500 ACE2-HEK293 cells/well were infected with 5ul per well of a mixture of SARS-CoV-2 Spike pseudotyped lentivirus (luciferase reporter) and iota-carrageenan polymer or carboxymethyl cellulose (CMC) polymer at varying polymer concentrations. Virus was incubated either solely with buffer (controls) or with buffer comprising different concentrations of iota-carrageenan or carboxymethyl cellulose (CMC) for 30 minutes prior to infection, i.e. 400, 133, 40, 13, 4, and 1.3 ug/ml iota-carrageenan, and 400, 40 and 4 ug/m of CMC. 24 hours after infection, the medium was changed to fresh HEK growth medium. 48 hours after infection the cells were lysed by freeze/thaw cycles before luciferase reagent was added to the wells to measure the luciferase activity. The infection rate or infection efficiency determined by measuring the luciferase activity indicates the percentage of infected cells in the presence of the antiviral protective carrageenan component, and thus the carrageenan component's protective efficacy against SARS-CoV-2 infection.

The results are depicted in Fig. 1, wherein maximum luciferase activity determined from cells infected with the virus in the absence of the protective carrageenan component (mock treatment) was set as 100% (= positive control), whereas the luciferase activity determined from uninfected cells was set as 0% (= negative control). Inhibition of infection by iota-carrageenan (black bars) was compared with the corresponding activity of carboxymethyl cellulose (CMC, hatched bars). Luciferase data were corrected for metabolic activity determined with Alamar blue using a parallel plate with identical set-up. Different polymer concentrations are indicated at the x-axis, and the corrected luciferase activity values indicating the percentage of infected ACE2-HEK293 cells are displayed on the y-axis. The data represent means of duplicates.

It can be taken from Fig. 1 that CMC had no infection inhibitory effect at 400 and 40 ug/ml, and a weak inhibitory effect at only 4 ug/ml. Whereas iota-carrageenan was highly effective at the highest concentration of 400 ug/ml resulting in a reduction of infection of more than 90%, relative to the unprotected positive control, while at concentrations of 133, 40 and 13 ug/ml iota-carrageenan still produced a sound inhibitory effect of approximately 80%, relative to the unprotected positive control. Only at the lowest concentrations of 4 and 1.3 ug/ml the infection inhibitory efficacy of iota-carrageenan seemed to fade away to some extent, even though at these concentrations iota-carrageenan was still able to keep the number of infected cells around 30% below the unprotected positive control.

It appears therefore that iota-carrageenan qualifies as a useful agent in the manufacture of a pharmaceutical composition or medicament for the prophylactic or therapeutic treatment of mammals, in particular human individuals, at risk of or suffering from coronavirus infection, and especially from infection with SARS CoV2.

### EXAMPLE 2: Protective Efficacy of Iota-Carrageenan Against SARS-CoV-2 at Varying Concentrations

The experimental setup of Example 1 was repeated with the following modifications:
7000 HEK293 cells expressing the ACE-2 receptor were infected with 750 infectious particles of the SARS-CoV-2 pseudotyped lentivirus expressing luciferase. Virus was incubated either solely with buffer (controls) or with buffer comprising different amounts of iota-carrageenan or hydroxypropylmethyl cellulose HPMC for 30 minutes prior to infection, i.e. 100, 33, 10, 3.3, 1 und 0.33 ug/ml of iota-carrageenan, and 100 ug/ml of HPMC (Fig. 2, x-axis). 24 h after infection the medium was replaced with fresh growth medium. 48 h after infection the cells were lysed by freeze/thaw-cycles, luciferase reagent was added and luciferase enzyme activity was determined and evaluated against the positve and negative controls.

In Fig. 2 the resulting values of infection rate (y-axis) were corrected for metabolic activity determined by Alamar blue on an identical plate. Cells mock-infected were set to 0%, while mock-treated infected cells were set to 100%. The bars represent means of triplicates with the standard deviation indicated.

It can be taken from Fig. 2 that iota-carrageenan is highly protective in a concentration range of from 100 ug/ml down to 10 ug/ml, whereas at very low concentrations of 1 ug/ml or less the inbibitory/protective efficacy against SARS-CoV-2 infection seems to fade away. It appears that there is a sound dose-dependent efficacy of iota-carrageenan.

HPMC applied at a single concentration of 100 ug/ml exerts a weak infection-inhibitory effect only.

### EXAMPLE 3: Comparision of Protective Efficacy of Various Negatively Charged Polymers Against Infection With SARS-CoV-2

The experimental setup of Example 2 was repeated with the following modifications:
In addition to iota-carrageenan also kappa-carrageenan, purified lambda-carrageenan, high molecular-weight (HMW) fucoidan, cellulose sulfate, heparan sulfate, and dextran sulfate were enrolled to the experimental setup at 3 different concentrations each, i.e. at 100 ug/ml, 10 ug/ml and 1 ug/ml. The results are depicted in Fig. 3.

Also, in order to better understand the foregoing protective activities of the polymers further polymeric agents have been tested under the same experimental conditions, i.e. carboxymethyl cellulose (CMC), hydroxypropylmethyl cellulose (HPMC), and galactose-4-sulfate (Gal-4-SO4), each of these polymers at a concentration of 100 ug/ml.

Surprisingly, while the carrageenans as well as cellulose sulfate and dextran sulfate exert highly protective efficacy at least at the highest concentrations, and particularly within a reange of from 100 to 10 ug/ml, fucoidan seems to be only poorly protective and heparan sulfate does not exert any protective effect at all.

Similarly, the comparative polymers CMC, HPMC, and Gal-4-SO4 are only poorly effective in the protection of the mammalian target cells against infection with modified lentivirus mimicking SARS-CoV-2.

The best results are achieved with iota- and lambda-carrageenan, and with cellulose sulfate and dextran sulfate. It shall be noted at this occasion that in previous studies on the antiviral efficacy of lambda-carrageenan, this polymer has been found to exert only poor antiviral efficacy, if at all. Most recent findings of the present inventors indicate that this lack of antiviral activity of lambda-carrageenan may have been due to the quality of commercially available lambda-carrageenan, which indeed contained only very low amounts, i.e. only up to about 5 % by weight, of lambda-carrageenan. Most of the material sold as "lambda carrageenan" was other polymers.

Lambda-carrageenan referred to herein and used in the Examples has been purified to contain at least 50 - 95%, preferably at least 75 % and typically around 90-95% by weight of all carrageenan and non-carrageenan polymeric fractions present in the respective specimens.

## Claims

1. A carrageenan component comprising iota-, kappa- or lambda-carrageenan, or any mixture of said carrageenans, for use as an antiviral active ingredient in a pharmaceutical composition or medicament for the prophylactic or therapeutic intervention of a SARS-CoV-2 infection in a mammal, especially in a human individual, wherein said carrageenan component is present in an antiviral effective amount and comprises at least 50% by weight, or at least 75% wt, or at least 95% wt of either iota-, kappa-, or lambda carrageenan, or of any mixture of said carrageenans, relative to total of carrageenans present in the composition or medicament.

2. The carrageenan component for the use as claimed in claim 1, wherein the lambda-carrageenan component is purified to comprise at least 50% by weight, or at least 75%, or 90 - 95% wt of lambda-carrageenan.

3. The carrageenan component for the use as claimed in claims 1 or 2, wherein the antiviral pharmaceutical composition or medicament is adapted for parenteral use and is preferably prepared as a skin lotion, cream, ointment, gel, powder, liquid solution, spray, foam, or a gargle solution.

4. The carrageenan component for the use as claimed in claim 1 or 2, wherein the composition is liquid or semi-solid and comprises as a ready-for-use preparation the carrageenan component in an amount of between 0.01 % and 10 %, preferably between 0.01 % and 5 %, most preferably between 0.1 % and 2 % by weight, relative to the total volume of the preparation.

5. The carrageenan component for the use as claimed in claim 1 or 2, wherein the composition is solid and comprises as a ready-for-use preparation the carrageenan-component in an amount of between 0.01 % and 10 %, preferably between 0.01 % and 5 %, most preferably between 0.1 % and 2 % by weight.

6. The carrageenan component for the use as claimed in any one of claims 1 to 5, wherein said composition further comprises at least one pharmaceutically acceptable carrier and/or additive, said pharmaceutically acceptable additive preferably selected from sodium and potassium chloride and being present in the composition in an amount of 1 % or less, preferably of 0.6 % or less.

7. The carrageenan component for the use as claimed in any one of claims 1 to 6, wherein the composition comprises a part or all of the carrageenans present by way of their salts, preferably by way of sodium salts.

8. The carrageenan component for the use as claimed in any one of claims 1 to 7, wherein the composition is attached by either coating or impregnation to a solid surface of a hygiene or sanitary item, particularly of a hygiene or sanitary glove, tissue or paper, especially a nasal tissue or paper, a cotton swab, dust mask or sanitary or medical facial mask.

9. The carrageenan component for the use as claimed in any one of claims 1 to 7, in combination with one or more non-carrageenan physiologically active substances typically applied in the prophylaxis or therapy of infectious and/or inflammatory diseases, allergies, and/or conditions of an impaired or suppressed immune system.

10. The carrageenan component for the use as claimed in any one of claims 1 to 9, wherein the human individual is a high-risk patient selected from the group consisting of a COPD-patient, an asthma patient, a person with allergies, a person with impaired immune, cardiac, or pulmonary system, and a transplantation patient.
